# EUROPEAN PATENT SPECIFICATION

(11) **EP 4 084 716 B1**
(45) Date of publication and mention of the grant of the patent: **30.07.2025**
(21) Application number: 20708608.3
(22) Date of filing: 29.01.2020
(51) Int. Cl.: A61B 18/14, A61B 18/00

(54) **USING IRRIGATION ON IRREVERSIBLE-ELECTROPORATION (IRE) ELECTRODES TO PREVENT ARCING**
VERWENDUNG VON IRRIGATION BEI ELEKTRODEN MIT IRREVERSIBLER ELEKTROPORATION (IRE) ZUR VERMEIDUNG VON LICHTBOGENBILDUNG
UTILISATION D'IRRIGATION SUR DES ÉLECTRODES D'ÉLECTROPORATION IRRÉVERSIBLE (IRE) POUR EMPÊCHER LA FORMATION D'ARCS

(30) Priority: 31.12.2019 US 201916731238
(43) Date of publication of application: 09.11.2022
(73) Proprietor: Biosense Webster (Israel) Ltd., Yokneam, 2066717 (IL)
(72) Inventor: ALTMANN, Andres Claudio, 2066717 Yokneam (IL); GOVARI, Assaf, 2066717 Yokneam (IL)
(74) Representative: Carpmaels & Ransford LLP
(86) International application number: PCT/IB2020/050681
(87) International publication number: WO 2021/136976

(56) References cited:
- US-A1- 2003 004 506
- US-A1- 2017 042 615
- US-A1- 2018 161 093
- US-A1- 2019 015 152
- US-A1- 2019 030 328
- US-A1- 2019 059 993
- US-A1- 2019 365 464

## Description

### FIELD OF THE INVENTION

The present invention relates generally to the invasive medical probes, and particularly to catheters for irreversible electroporation (IRE).

### BACKGROUND OF THE INVENTION

Delivery of high voltage irreversible electroporation (IRE) pulses to tissue was previously proposed in the patent literature. For example, U.S. Patent Application Publication 2011/0202052 describes a system that improves urine flow by increasing the inside diameter of the urethra going through the prostate by eroding the urethral wall, rather than by reducing the prostate volume. This is done by a specially designed IRE electrode, which limits the penetration depth of the electric field to the urethral wall. In an embodiment, a catheter carrying the electrodes can be made hollow and irrigation holes can be added. Such holes can be used to deliver a saline solution for cooling as well as medication to assist the procedure such as for pain reduction.

As another example, U.S. Patent Application Publication 2018/0296264 describes several devices and methods for treating obesity and diabetes by using electroporation to modulate the duodenal mucosa. In some embodiments, an endoscope is used that includes a lumen (e.g., an irrigation lumen) through which electrically conductive liquid can flow. When the electroporation device is in use, the electrically conductive liquid can flow through the lumen of the endoscope and thereafter reside in the duodenum between a proximal balloon and distal balloon inflated in the electroporation device. In this arrangement, energy from energized electrodes of the electroporation device can be conducted by the electrically conductive liquid to the duodenal mucosa, including within the crypts of the duodenal mucosa.

U.S. Patent 9,877,781 describes an electrode catheter device with indifferent electrode for direct current tissue therapies. An example of the catheter device has a flexible tubing with at least one ablation electrode. The catheter device may also be used with a sheath for introducing the flexible tubing inside a patient's body. An indifferent electrode on the sheath can provide a ground for a direct current (DC) pulse to deliver electrical energy and create an electrical field adjacent tissue. Various other embodiments are also disclosed. In one embodiment, of an irrigated catheter, the tip electrode and/or other locations in the tip assembly may be formed with a plurality of openings, e.g., between two or more of the electrodes. A central lumen may be in fluid communication with a fluid fitting on one end, and with the ports at the other end. Thus, an electricity conductive fluid or gel may be injected through the inner tube and secreted from the ports to form a current path and facilitate conduction of an electrical current between the electrodes.

US 2017/042615 A1 discloses a cardiac tissue ablation catheter including an inflatable and flexible toroidal or spherically shaped balloon disposed at a distal region of an elongate member.

US 2018/161093 A1 discloses an irrigated balloon catheter, including balloon carrying contact electrodes.

US 2019/365464 A1 discloses a balloon catheter including a shaft, a balloon made of an expandable membrane, a flexible substrate, one or more electrodes, and one or more radiopaque flags.

US 2019/015152 A1 discloses a reverse irrigation device comprising at least one ablation electrode and at least one reverse irrigation port.

US 2019/059993 A1 discloses a medical instrument including a shaft, multiple electrodes, and a vibration generator.

### SUMMARY OF THE INVENTION

The present invention provides a medical probe as defined in claim 1.

The scope of the present invention is defined by the appended claims.

In an embodiment, the irrigation channels are made of a thermally conducting material.

In another embodiment, the thermally conducting material comprises Nitinol.

There is additionally provided a system according to claim 4.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based irreversible electroporation (IRE) system, in accordance with an exemplary embodiment of the present disclosure;
Fig. 2 is an exploded perspective view of the irreversible electroporation (IRE) balloon catheter of Fig. 1, in accordance with an exemplary embodiment of the present disclosure; and
Fig. 3 is a side view of the assembled irreversible electroporation (IRE) balloon catheter of Fig. 2, in accordance with an exemplary embodiment of the present disclosure.

### DETAILED DESCRIPTION OF EMBODIMENTS

### OVERVIEW

Catheter-based irreversible electroporation (IRE) may be used to apply high-voltage bipolar pulses (e.g., between adjacent electrodes of the catheter) to achieve the high field strengths needed to destroy tissue cells to which the pulses are applied. For example, a medical probe, such as a balloon catheter, could be used to apply the high voltage pulses to tissue using a plurality of electrodes disposed on the balloon. Other types of catheters that carry electrodes, such as a basket catheter or a lasso catheter (made by Biosense-Webster, Irvine, California) may be used as well. However, the high voltages may cause arcing between electrodes applying the pulses, which causes drops in the electric field and may cause excessive heating.

Arcing may be generated when high-voltage pulses cause an electrode surface to heat and generate bubbles in blood in contact with electrodes, for example, by electrolysis. Due to the bubbles, the bipolar impedance increases and more gas may be formed around the electrode due to heating. The high voltage generates current through the high impedance gas, creating ionized plasma, and arcing occurs, usually at edges of the electrodes (e.g., edge locations over the perimeter of electrodes) where the current density is higher and temperature may locally peak.

Exemplary embodiments of the present disclosure that are described hereafter use irrigation to reduce the probability of arcing by locally cooling blood to suppress formation of gas bubbles. Since the arcing tends to occur on electrode edges, the irrigation is preferably concentrated in these regions.

Some exemplary embodiments provide a shaft for insertion into an organ of a patient, with a frame, where the frame can be an expandable frame or a fixed frame, coupled to a distal end of the shaft. The frame includes a plurality of electrodes disposed on an outer surface of the frame which are configured to apply IRE to tissue by applying voltage pulses. One or more irrigation channels are configured to flow irrigation fluid in a vicinity of the electrodes, to cool blood at edges of the electrodes.

In one exemplary embodiment, the irrigation is open (i.e., where the irrigation fluid, typically saline, is expelled from the IRE catheter into the patient) to remove heat from blood by heat convection. In another exemplary embodiment, the irrigation fluid runs in a closed circuit, where a cool fluid is recirculated from the catheter using tubing in thermal contact with the electrodes to remove heat from blood by heat conduction.

These irrigation methods, whether used separately or combined, significantly diminish heating of blood at electrode edges during application of IRE pulses to tissue, and by so doing prevent a buildup of conditions favorable to arcing.

Typically, a heat removal rate of few tens of milliwatts per second over the entire catheter should be sufficient. Due to the high heat capacity of water, heat removal by convection is readily achievable with an open irrigation that mixes cooler saline with blood at typical rates of at least several milliliters per minute. For example, irrigation can readily remove heat from radiofrequency balloon electrodes at a rate of several watts per electrode.

Thermal conduction, on the other hand, must be more carefully engineered, as heat removal rate by thermal conductivity of water is much lower, e.g., by about three orders of magnitude compared with convection. Still, proper design of the closed-circuit irrigation geometry and materials (e.g., avoiding plastic tubing in favor of materials, such as Nitinol, which have good thermal contact between tubing and electrode), as well as a lowered saline temperature to cool the tubes, can readily provide a sufficient rate of heat removal.

The disclosed open-circuit and closed-circuit irrigated IRE catheters enable the application of IRE treatment in a safe and electrically efficient manner, and may thus improve the clinical outcome of invasive IRE treatments, such as of an IRE treatment of cardiac arrhythmia.

### SYSTEM DESCRIPTION

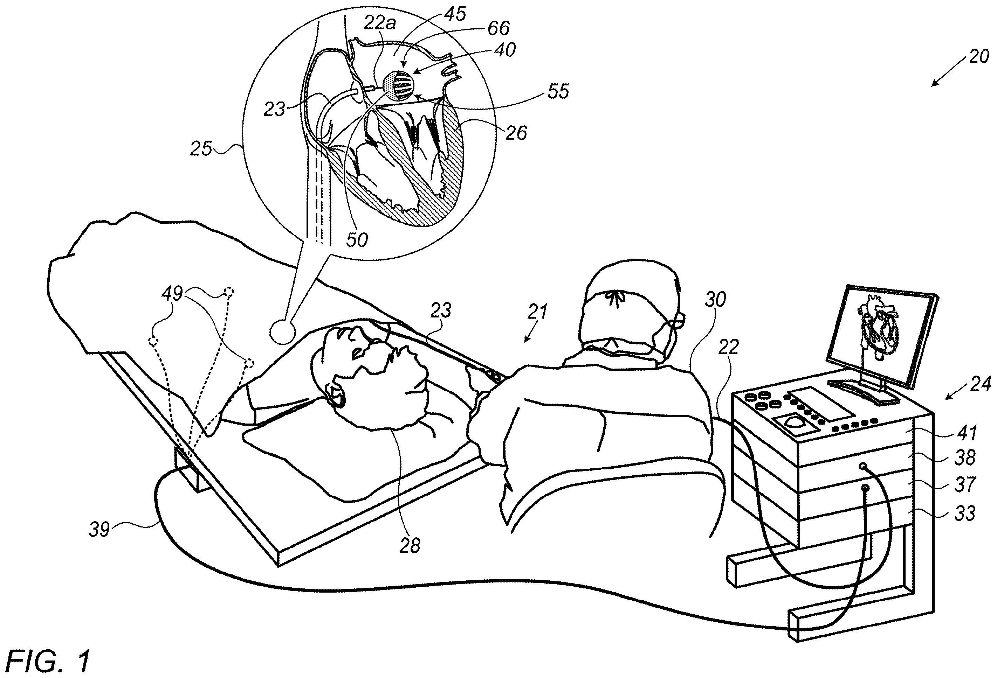

Fig. 1 is a schematic, pictorial illustration of a catheter-based irreversible electroporation (IRE) system 20, in accordance with an exemplary embodiment of the present disclosure. System 20 comprises a catheter 21, wherein a shaft 22 of the catheter is inserted into a heart 26 of a patient 28 through a sheath 23. The proximal end of catheter 21 is connected to a console 24.

Console 24 comprises an IRE generator 38 for applying IRE pulses via catheter 21 to irreversibly electroporate an ostium tissue of a pulmonary vein in left atrium 45 of heart 26. In the exemplary embodiment described herein, catheter 21 may be used for any other suitable therapeutic and/or diagnostic purpose, such as electrical sensing and/or irreversibly electroporating another tissue of heart 26.

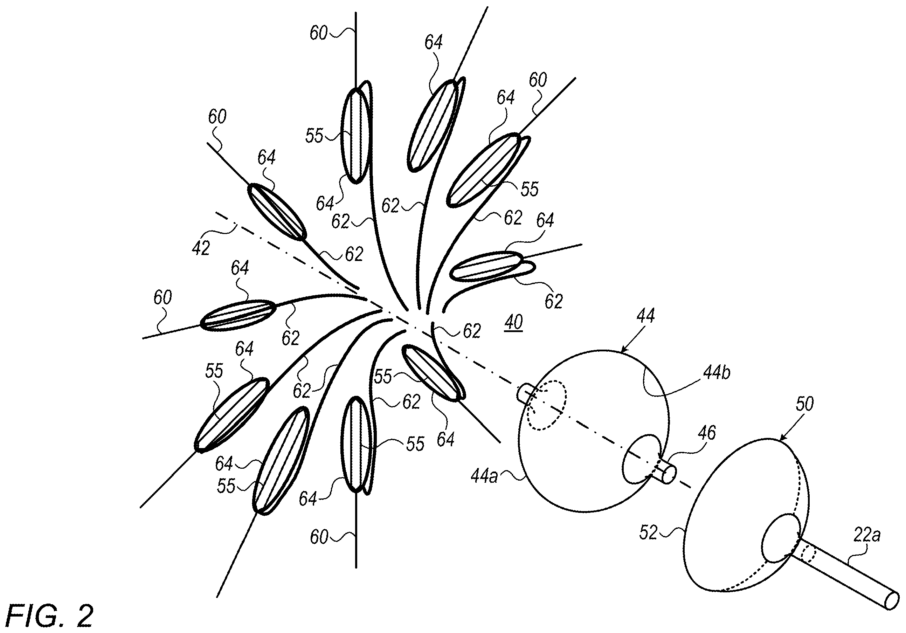

A physician 30 inserts shaft 22 through the vascular system of patient 28. As seen in inset 25, an expandable balloon catheter 40 that is fitted at a distal end 22a of shaft 22 comprises a high-voltage insulation cover membrane 50 in a form of a hemisphere and irrigation, further described in Fig. 2. Cover membrane 50 is described in U.S. Patent Application Serial No. 16/707175 filed December 9, 2019, entitled "Irreversible-Electroporation (IRE) Balloon Catheter with Membrane-Insulated High-Voltage Balloon wires," which is assigned to the assignee of the present patent application, which document is incorporated by reference herein.

During the insertion of shaft 22, balloon 40 is maintained in a collapsed configuration inside sheath 23. By containing balloon 40 in a collapsed configuration, sheath 23 also serves to minimize vascular trauma. Physician 30 navigates the distal end of shaft 22 to a target location in heart 26.

Once distal end 22a of shaft 22 has reached the target location, physician 30 retracts sheath 23 and expands balloon 40 by, for example, pumping saline into an internal volume defined by the aforementioned expendable membrane. Physician 30 then manipulates shaft 22 such that electrodes 55 disposed on balloon catheter 40 engage an interior wall of the ostium, and operates console 24 to apply high-voltage IRE pulses via electrodes 55 to the ostium tissue.

Console 24 comprises an irrigation pumping system 33 that pumps saline into balloon 40 via a pipe running inside shaft 22. Irrigation fluid pours out of irrigation holes 66 on the edges of electrodes 55 to cool blood by convection to avoid conditions favorable for arcing.

Console 24 comprises a processor 41, typically a general-purpose computer, with suitable front end and interface circuits 37 for receiving signals from catheter 21 and from external-electrodes 49, which are typically placed around the chest of patient 26. For this purpose, processor 41 is connected to external-electrodes 49 by wires running through a cable 39.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Although the illustrated exemplary embodiment relates specifically to the use of a balloon for IRE of heart tissue, the elements of system 20 and the methods described herein may alternatively be applied in controlling ablation using other sorts of multi-electrode ablation devices, such as ablation catheters having an expandable frame, e.g., a basket catheter, and catheters having a fixed frame, e.g., a Lasso^{®} catheter or a PentaRay^{®} catheter.

### USING IRRIGATION ON IRE ELECTRODES TO PREVENT ARCING

Fig. 2 is an exploded perspective view of irreversible electroporation (IRE) balloon catheter 40 of Fig. 1, in accordance with an exemplary embodiment of the present disclosure.

An expandable membrane 44 of balloon catheter 40 is attached to distal end 22a of shaft 22 at a proximal membrane portion 46 of membrane 44. Membrane 44 is disposed about a longitudinal axis 42 and has an outer surface 44a and an inner surface 44b. Outer surface 44a is exposed to the ambient environment while inner surface 44b is exposed to an internal volume of the balloon defined by membrane 44.

Expandable membrane 44 is configured to be expanded from a collapsed shape (generally an elongated tubular configuration) to a balloon (or generally spheroidal) shaped member. A plurality of electrodes 55 is disposed on outer surface 44a of the expandable membrane 44. Electrodes 55 are arranged equidistantly over a distal hemisphere portion of membrane 44. In the illustrated exemplary embodiment, each of electrodes 55 has an insulated electrical wire 60, which is electrically connected to conduct high voltage to the electrode. Electrical wires 60 are coupled to the output of IRE generator 24 by wiring (not shown) that goes to console 24 via hollow shaft 22.

The underside surface of each electrode 55 is not exposed to the ambient environment and is typically bonded to outer surface 44a of membrane 44.

An expandable cover membrane 50, having a border 52, encapsulates wires 60 between cover membrane 50 and expandable membrane 44 so that wires 60 are constrained between membrane 44 and cover membrane 50. In this way, wires 60 are resilient to dielectric breakdown due to high voltage electrical signals that they conduct during an IRE procedure.

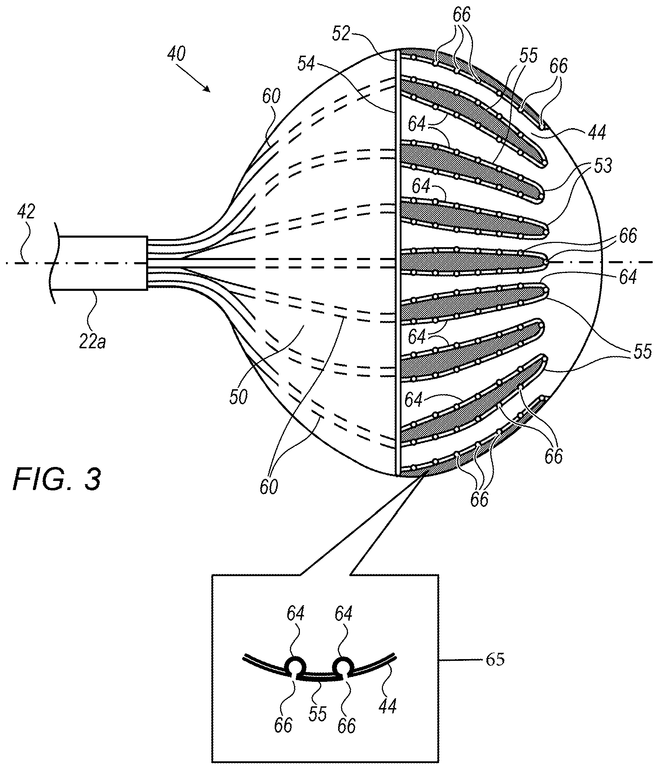

Each electrode 55 has a tube 62 that feeds a circulation tube 64 encircling the electrode. In some exemplary embodiments, circulation tubes 64 are fitted with irrigation holes, as described in Fig. 3, to cool electrode edges by convection. In other exemplary embodiments, circulation tubes 64 are made of a heat-conductive material, such as Nitinol, and each circulation tube 64 is thermally coupled to a respective electrode to cool the edges of the electrode by heat conduction.

Fig. 3 is a side view of the assembled irreversible electroporation (IRE) balloon catheter 40 of Fig. 2, in accordance with an exemplary embodiment of the present disclosure. As seen, each of the plurality of electrodes 55 defines an area not covered by expandable cover membrane 50 to allow the electrodes to be exposed to the ambient environment.

The plurality of electrodes 55 is disposed equiangularly about longitudinal axis 42, such that cover membrane 50 encapsulates a proximal edge of each electrode 55. Typically, each electrode 55 is coupled to the outer surface of expandable membrane 44 via a substrate 53 which itself is connected, or bonded, to the outer surface of membrane 44.

As can be seen in Fig. 3, each tube 62 runs within the internal volume of balloon 40 (under membrane 44) and extends from distal end 22a to a respective circulation tube 64 of electrode 55 such that each tube 62 substantially follows the topography of membrane 44. In this way there is little or no risk of the tubes being entangled during the expansion and collapse of balloon 40.

In the illustrated exemplary embodiment, further detailed in cross-sectional inset 65, the irrigated fluid of each circulation tube 64 flows via irrigation holes 66 to the outside of balloon 40, i.e., into the ambient environment, to cool the blood at the edge of electrode 55.

The exterior wall of membrane 44 and tubes (62, 64) are made of bio-compatible materials, for example, formed from a plastic (e.g., polymer) such as polyethylene terephthalate (PET), polyurethane, or PEBAX^{®}.

Any of the examples or embodiments described herein may include various other features in addition to or in lieu of those described above. In particular, the simplified configurations shown in Figs. 2 and 3 are chosen purely for the sake of conceptual clarity and simplicity of presentation. For example, electrodes 55 may be disposed with temperature sensors.

Although the embodiments described herein mainly address cardiac applications, the methods and systems described herein can also be used in other medical applications, such as in neurology and otolaryngology.

It will thus be appreciated that the embodiments described above are cited by way of example, and that the present invention is not limited to what has been particularly shown and described hereinabove. Rather, the scope of the present invention is defined by the appended claims.

## Claims

1. A medical probe for irreversible electroporation with irrigation, the medical probe comprising:
a shaft (22) for insertion into an organ of a patient; and
a frame coupled to a distal end of the shaft, the frame comprising:
a plurality of electrodes (55) disposed on an outer surface of the frame and configured to apply irreversible electroporation (IRE) to tissue by applying voltage pulses; and
one or more irrigation channels (64), configured to flow irrigation fluid in a vicinity of the electrodes, wherein the irrigation channels (64) are thermally coupled to the electrodes (55), and are configured to flow the irrigation fluid in a closed circuit to remove heat from edges of the electrodes.

2. The medical probe according to claim 1, wherein the irrigation channels (64) are made of a thermally conducting material.

3. The medical probe according to claim 2, wherein the thermally conducting material comprises Nitinol.

4. A system comprising the medical probe of any preceding claim, and further comprising a generator, configured to generate IRE pulses, and to apply the IRE pulses between adjacent ones of the electrodes, so as to electroporate the tissue.

## Patentansprüche

1. Medizinische Sonde für irreversible Elektroporation mit Spülung, die medizinische Sonde umfassend:
einen Schaft (22) für ein Einführen in ein Organ eines Patienten; und
einen Rahmen, der mit einem distalen Ende des Schafts gekoppelt ist, der Rahmen umfassend:
eine Vielzahl von Elektroden (55), die auf einer Außenoberfläche des Rahmens angeordnet und konfiguriert sind, um durch Anlegen von Spannungsimpulsen eine irreversible Elektroporation (IRE) an Gewebe durchzuführen; und
einen oder mehrere Spülkanäle (64), die konfiguriert sind, um eine Spülflüssigkeit in eine Nähe der Elektroden fließen zu lassen, wobei die Spülkanäle (64) mit den Elektroden (55) thermisch gekoppelt und konfiguriert sind, um die Spülflüssigkeit in einem geschlossenen Kreislauf fließen zu lassen, um Wärme von Kanten der Elektroden abzuleiten.

2. Medizinische Sonde nach Anspruch 1, wobei die Spülkanäle (64) aus einem wärmeleitenden Material hergestellt sind.

3. Medizinische Sonde nach Anspruch 2, wobei das wärmeleitende Material Nitinol umfasst.

4. System, umfassend die medizinische Sonde nach einem der vorstehenden Ansprüche und ferner umfassend einen Generator, der konfiguriert ist, um IRE-Impulse zu erzeugen und um die IRE-Impulse zwischen angrenzenden der Elektroden anzulegen, um das Gewebe zu elektroporieren.

## Revendications

1. Sonde médicale pour l'électroporation irréversible avec irrigation, la sonde médicale comprenant :
un arbre (22) destiné à être inséré dans un organe d'un patient ; et
un cadre accouplé à une extrémité distale de l'arbre, le cadre comprenant :
une pluralité d'électrodes (55) disposées sur une surface extérieure du cadre et configurées pour appliquer une électroporation irréversible (IRE) au tissu en appliquant des impulsions de tension ; et
un ou plusieurs canaux d'irrigation (64), configurés pour faire circuler un liquide d'irrigation à proximité des électrodes, dans laquelle les canaux d'irrigation (64) sont couplés thermiquement aux électrodes (55) et sont configurés pour faire circuler le liquide d'irrigation en circuit fermé afin d'éliminer la chaleur des bords des électrodes.

2. Sonde médicale selon la revendication 1, dans laquelle les canaux d'irrigation (64) sont constitués d'un matériau thermoconducteur.

3. Sonde médicale selon la revendication 2, dans laquelle le matériau thermoconducteur comprend du Nitinol.

4. Système comprenant la sonde médicale selon l'une quelconque revendication précédente, et comprenant en outre un générateur, configuré pour générer des impulsions IRE, et pour appliquer les impulsions IRE entre les électrodes adjacentes, de manière à électroporter le tissu.
